# EUROPEAN PATENT APPLICATION

(11) **EP 4 524 252 A1**
(43) Date of publication of application: **19.03.2025**
(21) Application number: 23803542.2
(22) Date of filing: 08.05.2023
(51) Int. Cl.: C12P 19/04, C08B 37/00

(54) **METHOD FOR PRODUCING SIALIC-ACID-CONTAINING SUGAR CHAIN**

(30) Priority: 13.05.2022 JP 2022079634
(71) Applicant: Glytech, Inc., Kyoto-shi, Kyoto 600-8813 (JP)
(72) Inventor: YAMAMOTO, Takahiro, Kyoto-shi, Kyoto 600-8813 (JP); MURASE, Takefumi, Kyoto-shi, Kyoto 600-8813 (JP); MATSUO, Satoko, Kyoto-shi, Kyoto 600-8813 (JP); NISHIUCHI, Yuji, Kyoto-shi, Kyoto 600-8813 (JP); ISHII, Kazuyuki, Kyoto-shi, Kyoto 600-8813 (JP); OCHIAI, Hirofumi, Kyoto-shi, Kyoto 600-8813 (JP); NOGUCHI, Masato, Kyoto-shi, Kyoto 600-8813 (JP)
(74) Representative: Weickmann & Weickmann PartmbB
(86) International application number: PCT/JP2023/017322
(87) International publication number: WO 2023/219065

(57) **Abstract**

[Problem] The purpose of the present invention is to provide a method of manufacturing a sugar chain-containing composition having higher purity (uniformity) from chicken egg. [Solution] Provided is a method of manufacturing a composition comprising sugar chains having α2,6-linked sialic acids at all non-reducing ends at high purity from a chicken egg-derived sugar chain extract. The method is characterized by performing a treatment for enriching sugar chains having α2,6-linked sialic acids at all non-reducing ends in chicken egg-derived sugar chain extract.

## Description

### Technical Field

The present disclosure relates to a manufacturing method of sialic acid-containing sugar chain, and more specifically, to a method of manufacturing a composition comprising sugar chains having α2,6-linked sialic acids at all non-reducing ends at high purity from chicken egg. The present disclosure also relates to a composition comprising sugar chains having α2,6-linked sialic acids at all non-reducing ends at high purity.

### Background Art

Sugar chains have been clearly shown to play various roles in the living body, and are employed for modification of pharmaceutical active ingredients, manufacturing of pharmaceuticals, as well as for assays etc. High uniformity is demanded for sugar chains employed for such use in view of quality or precision.

Up until now, as one of the manufacturing methods of sugar chains, a method of isolation and purification from glycoproteins that exist in chicken eggs has been known (Non-Patent Literature 1, Patent Literatures 1 and 2).

### Citation List

### Patent Literatures

[Patent Literature 1] Japanese Unexamined Patent Application Publication No. 2003-128703
[Patent Literature 2] Japanese Unexamined Patent Application Publication No. 2011-162762

### Non-Patent Literatures

[Non-Patent Literature 1]Liu et al., "Improved isolation and characterization procedure of sialylglycopeptide from egg yolk powder", Carbohydrate Research 452 (2017) 122-128

### Summary of the Invention

### Problems to be Solved by the Invention

The object of the present disclosure is to provide a method of manufacturing a sugar chain-containing composition having higher purity (uniformity) from chicken egg.

### Means for Solving the Problems

Until now, it has been thought that sialic acids on the non-reducing ends of sugar chains manufactured from chicken eggs are all α2,6-linked. The inventors of the present invention have recently found, by employing analysis that is different from those conventionally and commonly employed, that a sugar chain mixture extracted from chicken egg comprises sugar chains having α2,3-linked sialic acids at a portion or all of the non-reducing ends as a trace amount of impurity, thus coming to complete the present invention.

The present invention encompasses the following characteristics:
(1) A method of manufacturing a composition comprising sugar chains having α2,6-linked sialic acids at all non-reducing ends at high purity from a chicken egg-derived sugar chain extract, characterized by performing a treatment for enriching sugar chains having α2,6-linked sialic acids at all non-reducing ends in said chicken egg-derived sugar chain extract.
(2) The manufacturing method according to (1), wherein said sugar chain is a disialo sugar chain.
(3) The manufacturing method according to (1), wherein said sugar chain is in the form of the following compound: 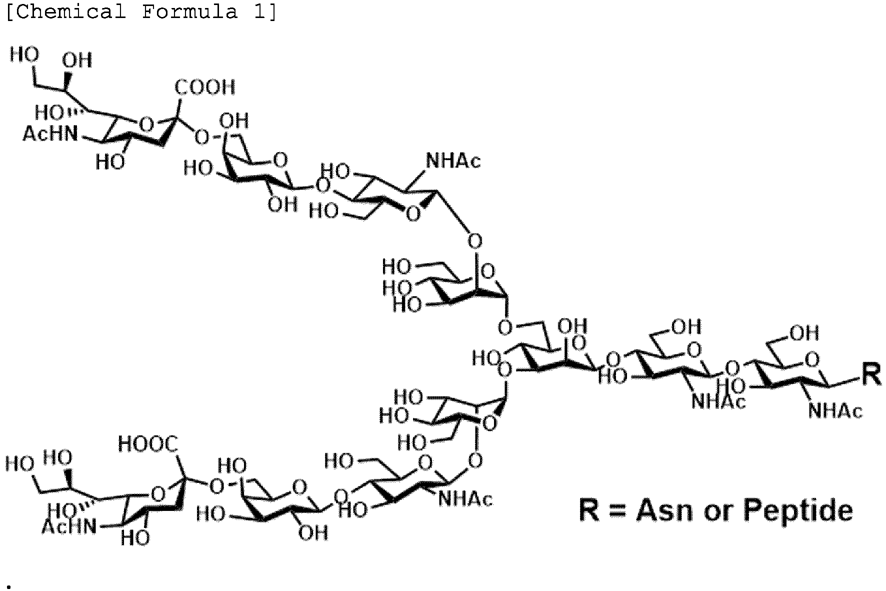
(4) The manufacturing method according to (1), wherein said enrichment treatment comprises separating sugar chains having α2,6-linked sialic acids at all non-reducing ends from sugar chains having α2,3-linked sialic acid at any non-reducing end.
(5) The manufacturing method according to (4), wherein said separation is performed by liquid chromatography.
(6) The manufacturing method according to (1), wherein said enrichment treatment comprises performing enzyme treatment or treatment by acid hydrolysis on sugar chains having α2,3-linked sialic acid at any non-reducing end.
(7) The manufacturing method according to (6), wherein said enzyme treatment comprises treatment by α2,3-sialidase.
(8) The manufacturing method according to (1), wherein said enrichment treatment comprises derivatization of sugar chains having α2,6-linked sialic acids at all non-reducing ends or sugar chains having α2,3-linked sialic acid at any non-reducing end.
(9) The manufacturing method according to (1), further comprising introducing a lipophilic substituent into the sugar chain.
(10) The manufacturing method according to (9), wherein the introduction method of the lipophilic substituent is esterification, thioesterification, amidation, or hydrazidation of the sialic acid carboxy group.
(11) The manufacturing method according to (9), wherein the lipophilic substituent is a benzyl group introduced into the carboxylic acid of sialic acid.
(12) A composition manufactured by the manufacturing method according to any one of (1) to (11).
(13) A composition consisting essentially of sugar chains having α2,6-linked sialic acids at all non-reducing ends.
(14) The composition according to (13), wherein said sugar chain is a disialo sugar chain.
(15) The manufacturing method according to (13), wherein said sugar chain is in the form of the following compound: 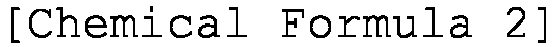 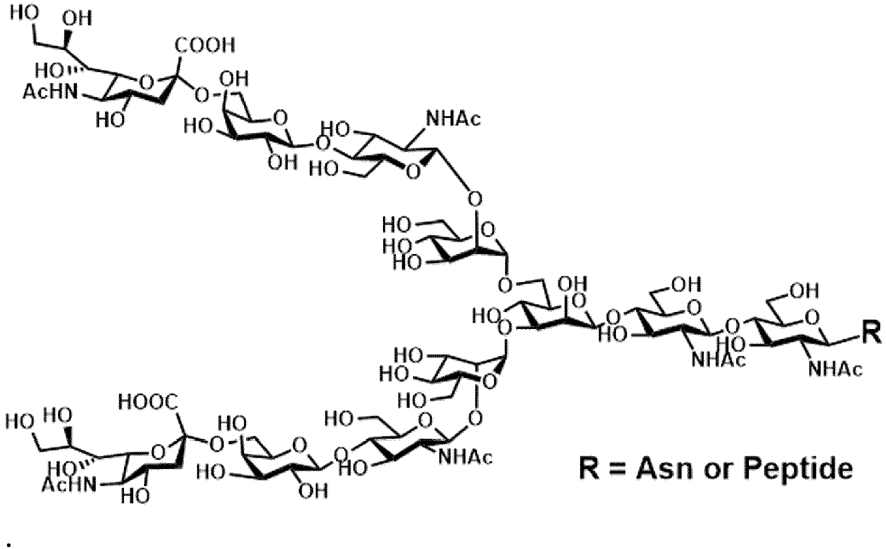
(16) The composition according to (13), wherein at least 97% of all sugar chains are sugar chains having α2,6-linked sialic acids at all non-reducing ends.
(17) The composition according to (13), wherein at least 98% of all sugar chains are sugar chains having α2,6-linked sialic acids at all non-reducing ends.
(18) The composition according to (13), wherein at least 99% of all sugar chains are sugar chains having α2,6-linked sialic acids at all non-reducing ends.
(19) The composition according to (13), wherein
   at least 95% of all sugar chains are sugar chains having α2,6-linked sialic acids at all non-reducing ends, and
   it comprises sugar chains having α2,3-linked sialic acid at any non-reducing end at less than 2% of all sugar chains.
(20) A method of manufacturing a composition comprising sugar chains having α2,6-linked sialic acids at all non-reducing ends at high purity from a sugar chain mixture comprising sugar chains having α2,6-linked sialic acids at all non-reducing ends and sugar chains having α2,3-linked sialic acid at any non-reducing end, characterized by purifying said sugar chains in said sugar chain mixture by the following:
   (a) subjecting to liquid chromatography employing a HILIC column
   (b) degrading or denaturing sugar chains having α2,3-linked sialic acid on any of the non-reducing ends, and then subjecting to liquid chromatography; or
   (c) derivatizing sugar chains having α2,6-linked sialic acids at all non-reducing ends, and then subjecting to liquid chromatography.
(21) The manufacturing method according to (20), wherein said sugar chain is a disialo sugar chain.
(22) The manufacturing method according to (20), wherein said sugar chain is in the form of the following compound: 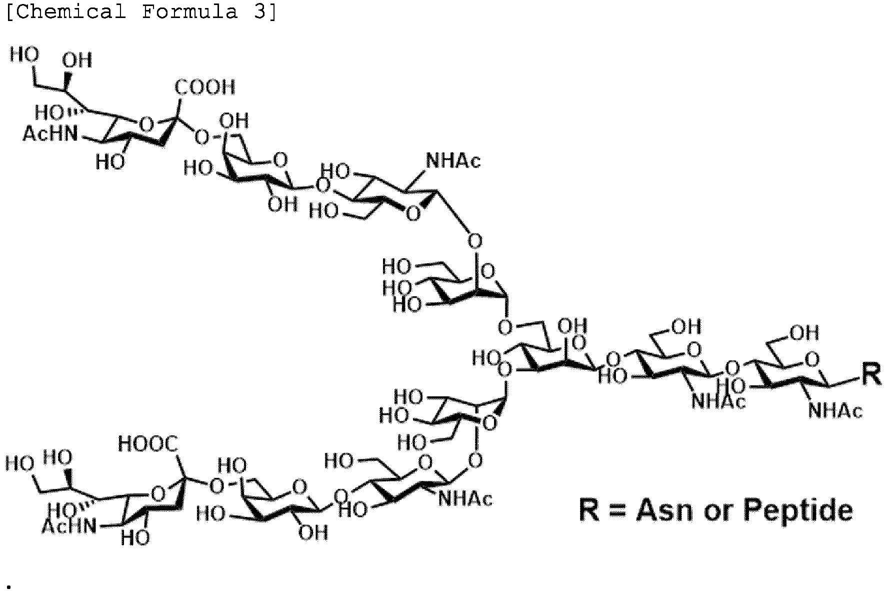
(23) The manufacturing method according to (20), wherein said degradation comprises performing enzyme treatment or treatment by acid hydrolysis on sugar chains having α2,3-linked sialic acid at any non-reducing end.
(24) The manufacturing method according to (23), wherein said enzyme treatment comprises treatment by α2,3-sialidase.
(25) The manufacturing method according to (20), wherein said denaturing comprises derivatizing any of sugar chains having α2,6-linked sialic acids at all non-reducing ends and sugar chains having α2,3-linked sialic acid at any of the non-reducing ends, or both.

Those skilled in the art will be able to recognize that an invention of any combination of one or more characteristics of the present invention described above is also encompassed by the scope of the present invention.

### Effects of the Invention

According to the present disclosure, a method of manufacturing a sugar chain-containing composition having higher purity (uniformity) from chicken egg is provided.

### Brief Description of the Drawings

Figure 1 shows the result of analysis of disialo sugar chain asparagine mixture. The top figure shows the result of analyzing the disialo sugar chain asparagine-Fmoc mixture obtained and prepared from chicken egg according to Reference Example 2 with HPLC in HILIC mode. The bottom figure shows the mass spectrometry result (ESI-MS) of the minor peak (3.7%) found by HPLC in HILIC mode.
Figure 2 shows the result of HPLC analysis of the minor peak found in Figure 1.
Figure 3 shows the result of HPLC in HILIC mode of the disialo sugar chain asparagine-Fmoc mixture obtained in Reference Example 2 and the impurity obtained in Example 2 (left figure), as well as the result of HPLC in ODS mode of the impurity found by the aforementioned HPLC (right figure).
Figure 4 each show the results of HPLC analysis of the impurities obtained in Example 2. In the figure, the vertical axis is the absorbance (mAU) and the horizontal axis is time (minutes).
Figure 5 shows the result of HPLC analysis of the enzyme treatment (α2,3-sialidase or α2,3-2,6-2,8-sialidase) reaction product of the impurity obtained in Example 2.
Figure 6 shows the result of HPLC analysis of the enzyme treatment (α2,3-sialidase or α2,3-2,6-2,8-sialidase) reaction product of GT-25001.
Figure 7 shows the result of HPLC analysis of the enzyme treatment (α2,3-sialidase or α2,3-2,6-2,8-sialidase) reaction product of GT-25025.
Figure 8 shows comparison of amounts of impurity between lots of disialo sugar chain asparagine-Fmoc mixtures prepared from delipidated egg yolk.
Figure 9 shows the HPLC result of the enzyme treatment (α2,3-sialidase) reaction product of the disialo sugar chain asparagine-Fmoc mixture obtained and prepared from chicken egg according to Reference Example 2.
Figure 10 shows the HPLC results of the enzyme treatment (α2,3-sialidase) reaction product of the disialo sugar chain asparagine-Fmoc mixture obtained and prepared from chicken egg according to Reference Example 2, and of after purification thereof.
Figure 11 shows the HPLC result after condensation reaction of the disialo sugar chain asparagine-Fmoc mixture obtained and prepared from chicken egg according to Reference Example 2.
Figure 12 shows the HPLC result of the reaction product of after Bn-ation treatment, deprotection of Bn and Fmoc groups, and then Fmoc-ation again of the disialo sugar chain asparagine-Fmoc mixture obtained and prepared from chicken egg according to Reference Example 2.
Figure 13 shows the HPLC result of the reaction product of enzyme treatment (α2,3-sialidase) of commercially available SGP having the sugar chain cleaved and labeled.

### Description of Embodiments

### 1. Introduction

The present disclosure, in one aspect, relates to a method of manufacturing a composition comprising sugar chains having α2,6-linked sialic acids at all non-reducing ends at high purity from a chicken egg-derived sugar chain extract (hereinafter also referred to as "the manufacturing method (1) of the present disclosure").

The present disclosure, in another aspect, relates to a method of manufacturing a composition comprising sugar chains having α2,6-linked sialic acids at all non-reducing ends at high purity from a sugar chain mixture comprising sugar chains having α2,6-linked sialic acids at all non-reducing ends and sugar chains having α2,3-linked sialic acid at any non-reducing end (hereinafter also referred to as "the manufacturing method (2) of the present disclosure").

The present disclosure, in yet another aspect, relates to a composition manufactured by the method according to the manufacturing method (1) or (2) of the present invention.

The present disclosure, in yet another aspect, relates to a composition consisting essentially of sugar chains having α2,6-linked sialic acids at all non-reducing ends.

In the present disclosure, the "chicken egg-derived sugar chain extract" is a fraction consisting of particular sialyl sugar chains extracted/separated from chicken egg, preferably its egg yolk. The "chicken egg-derived sugar chain extract" is typically a fraction consisting of disialo sugar chains.

Sugar chains contained in the "chicken egg-derived sugar chain extract" can be extracted in any form such as free sugar chain, glycoamino acid, and glycopeptide, and those skilled in the art can appropriately select the desired form.

In one embodiment of the present disclosure, the "chicken egg-derived sugar chain extract" is obtained mainly in the form of sialylglycopeptide (SGP). In such embodiments, in the sugar chain extraction process from chicken egg, treatment by a peptidase is not performed.

In another embodiment of the present disclosure, the "chicken egg-derived sugar chain extract" is obtained mainly in the form of sugar chain asparagine. In such embodiments, in the sugar chain extraction process from chicken egg, treatment by a peptidase is performed.

In the present disclosure, the "chicken egg-derived sugar chain extract" refers to a sugar chain-containing composition obtained by any means that can be employed for obtaining a sugar chain compound from chicken egg (preferably egg yolk). Means that can be employed for obtaining a sugar chain compound from chicken egg (preferably egg yolk) can include, but are not limited to, for example, means described in e.g. the above Patent Literatures 1 and 2 as well as Non-Patent Literature 1 which are incorporated herein by reference.

The "chicken egg-derived sugar chain extract" can be exemplarily obtained by phenol extraction against raw egg yolk, and a subsequent purification process employing six size exclusion and ion exchange columns (A. Seko et al., Biochim. Biophys. Acta-Gen. Subj. 1335(1997) 23.). In the above process, porous graphite carbon column (PGC) column chromatography can also be employed instead of the size exclusion chromatography (Y. Zou et al., J. Carbohydr. Chem. 31(2012) 436.). Moreover, the above process can be optimized by lipid removal via diethyl ether extraction (B. Sun et al., Carbohydr. Res. 396(2014) 62).

Moreover, extraction by aqueous acetone solution of raw egg yolk, and a subsequent activated carbon column purification can also be employed for obtaining the "chicken egg-derived sugar chain extract".

Moreover, diethyl ether washing of egg yolk powder and extraction by aqueous ethanol solution, and a subsequent activated carbon column purification can also be employed for obtaining the "chicken egg-derived sugar chain extract" (L. Liu et al., Carbohydrate. Res. 452(2017) 122).

Moreover, extraction by delipidated egg yolk powder aqueous solution, and a subsequent ODS purification can also be employed for obtaining the "chicken egg-derived sugar chain extract" (Japanese Unexamined Patent Application Publication No. 2011-162762).

### 2. Manufacturing method (1)

The manufacturing method (1) of the present disclosure is characterized by performing a treatment for enriching sugar chains having α2,6-linked sialic acids at all non-reducing ends in said chicken egg-derived sugar chain extract.

In one embodiment, the sugar chain enriched by the manufacturing method (1) of the present disclosure is in the following form:

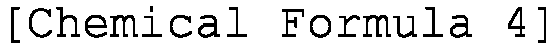

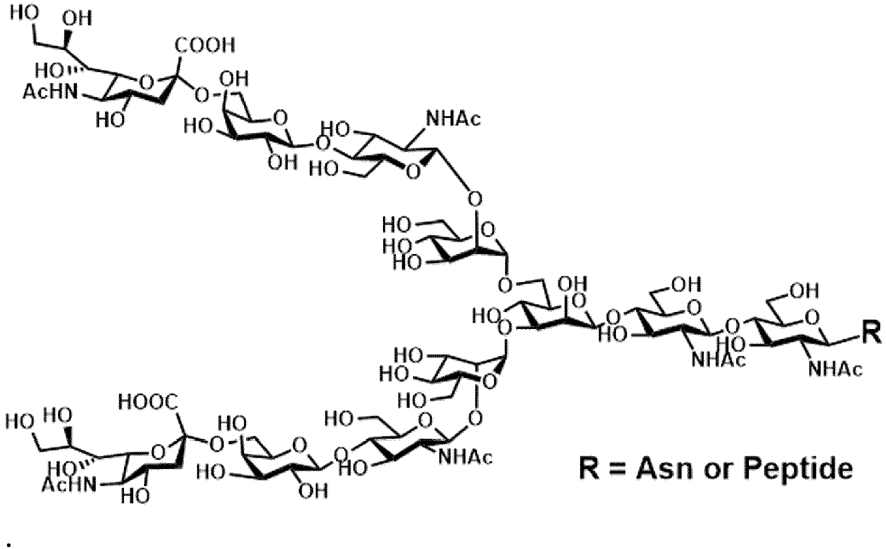

In the manufacturing method (1) of the present disclosure, the enrichment of sugar chains having α2,6-linked sialic acids at all non-reducing ends are performed by separation from sugar chains other than said sugar chains. Said separation is not particularly limited as long as it is a means that can separate substances based on molecular weight, charge, and other chemical natures, but liquid column chromatography is preferably employed.

In one embodiment of the present disclosure, the enrichment of sugar chains having α2,6-linked sialic acids at all non-reducing ends are performed by a treatment comprising separating sugar chains having α2,6-linked sialic acids at all non-reducing ends from sugar chains having α2,3-linked sialic acid at any non-reducing end. Sugar chains having α2,6-linked sialic acids at all non-reducing ends and sugar chains having α2,3-linked sialic acid at any non-reducing end are only different in the binding mode of sugar chains at non-reducing ends, and molecular weights are also identical. The inventors of the present invention have recently found that a mixture of both sugar chains can be separated by liquid chromatography employing a HILIC column. Accordingly, in one embodiment, chromatography employing liquid HILIC column is employed for the separation of sugar chains having α2,6-linked sialic acids at all non-reducing ends and sugar chains having α2,3-linked sialic acid at any non-reducing end.

In another embodiment, before subjecting to treatment for separation, a treatment for degrading sugar chains having α2,3-linked sialic acid at any non-reducing end is performed. Said degradation includes, for example, a treatment for specifically degrading α2,3-linked sialic acids by an enzyme or acid hydrolysis. Enzymes that can be used for said enzyme treatment can include, but are not limited to, utilization of α2,3-sialidase, non-specific sialidase, sialyltransferase having sialidase activity, and the like.

In another embodiment, before subjecting to treatment for separation, a treatment for denaturing sugar chains having α2,3-linked sialic acid at any non-reducing end is performed. Said denaturing can include, for example, introduction of a substituent by e.g. lactonization, as well as esterification, thioesterification, amidation, hydrazidation, and the like of the α2,3-linked sialic acid. α2,3-linked sialic acid is known to form a lactone in the molecule when the carboxylic acid at position 1 of the sialic acid is activated (Glycoconj. J. (2021) 38: 157-166), and the lactonization utilizes this property.

In another embodiment, before subjecting to treatment for separation, a treatment for derivatizing sugar chains having α2,6-linked sialic acids at all non-reducing ends is performed. Said derivatization can include, for example, esterification, thioesterification, amidation, hydrazidation, and the like of the sialic acid carboxy group. The derivatized sugar chain may be utilized as is, or a step of removing the substituent may be included.

Pretreatment before separation such as above has an advantage in that it facilitates the separation of α2,6-linked sialic acid, and that it can use ordinary columns suitable for mass production.

### 3. Manufacturing method (2)

The manufacturing method (2) of the present disclosure is characterized by purifying said sugar chains in the sugar chain mixture by the following:
(a) subjecting to liquid chromatography employing a HILIC column;
(b) degrading or denaturing sugar chains having α2,3-linked sialic acid on any of the non-reducing ends, and then subjecting to liquid chromatography; or
(c) derivatizing sugar chains having α2,6-linked sialic acids at all non-reducing ends, and then subjecting to liquid chromatography.

The sugar chain mixture in the aforementioned embodiment is not particularly limited as long as it is a sugar chain mixture comprising sugar chains having α2,6-linked sialic acids at all non-reducing ends and sugar chains having α2,3-linked sialic acid at any non-reducing end, and includes any sugar chain extract that is extracted from chicken egg or its egg yolk, as well as a mixture of sugar chains manufactured by chemical synthesis, a mixture of sugar chains cultured by gene recombination technology, and the like.

In one embodiment, the sugar chain purified by the manufacturing method (2) of the present disclosure is in the following form:

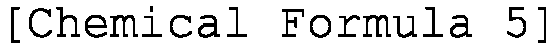

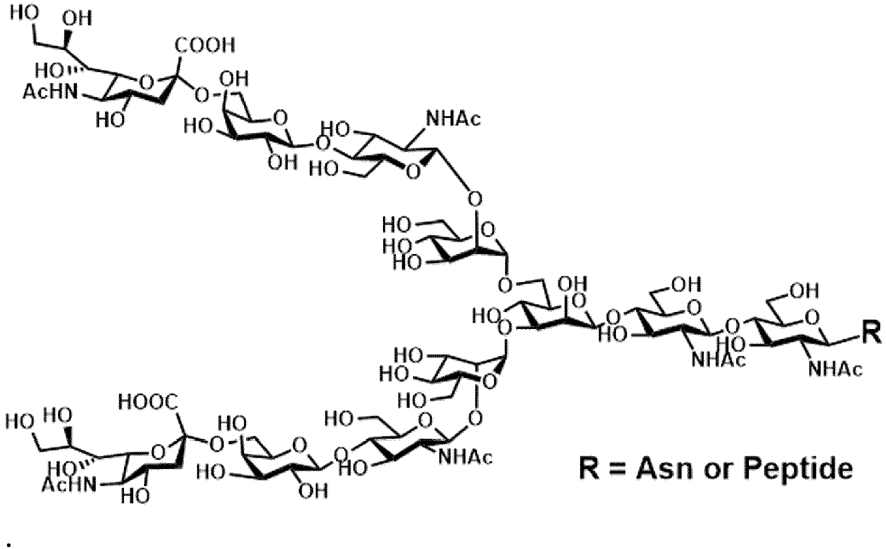

Said degradation includes, for example, a treatment for preferentially and specifically degrading α2,3-linked sialic acid by an enzyme. Enzymes that can be used for said enzyme treatment can include, but are not limited to, utilization of α2,3-sialidase, non-selective sialidase, sialyltransferase having sialidase activity, and the like.

In another embodiment, before subjecting to treatment for separation, a treatment for denaturing sugar chains having α2,3-linked sialic acid at any non-reducing end is performed. Said denaturing can include, for example, lactonization, esterification, thioesterification, amidation, hydrazidation, and the like of the α2,3-linked sialic acid.

In another embodiment, before subjecting to treatment for separation, a treatment for derivatizing sugar chains having α2,6-linked sialic acid at non-reducing ends is performed. Said derivatization can include, for example, esterification, thioesterification, amidation, hydrazidation, and the like of the sialic acid carboxy group. The derivatized sugar chain may be utilized as is, or a step of removing the substituent may be included.

In one embodiment of the present disclosure, the manufacturing method (1) or (2) of the present disclosure further comprises introducing a lipophilic substituent into the enriched or purified sugar chain. Said introduction of a substituent is typically introduction into the carboxylic acid group of the sialic acid. Sugar chains can thereby be stabilized. Said introduction of a substituent into the carboxylic acid group of the sialic acid is within the technical scope of those skilled in the art.

In one embodiment, the introduction method of the lipophilic substituent that can be used for the present disclosure can include esterification, thioesterification, amidation, hydrazidation, and the like of the sialic acid carboxy group.

In another embodiment, the lipophilic substituents that can be used for the present disclosure can include, but are not limited to, a benzyl group and a phenacyl group.

### 4. Composition

The composition manufactured by the above manufacturing method is a composition consisting essentially of sugar chains having α2,6-linked sialic acids at all non-reducing ends.

In one embodiment of the present disclosure, "consists essentially of" may mean that among all sugar chains contained in the composition, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% are sugar chains having α2,6-linked sialic acids at all non-reducing ends.

In another embodiment of the present disclosure, "consists essentially of" may mean that among all sugar chains contained in the composition, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% are sugar chains having α2,6-linked sialic acids at all non-reducing ends, and sugar chains having α2,3-linked sialic acid at any non-reducing end are less than 2.5%, less than 2.4%, less than 2.3%, less than 2.2%, less than 2.1%, less than 2.0%, less than 1.9%, less than 1.8%, less than 1.7%, less than 1.6%, less than 1.5%, less than 1.4%, less than 1.3%, less than 1.2%, less than 1.1%, less than 1.0%, less than 0.9%, less than 0.8%, less than 0.7%, less than 0.6%, less than 0.5%, less than 0.4%, less than 0.3%, less than 0.2%, or less than 0.1% of all sugar chains.

Note that the terms used herein are to be employed to describe particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, should be construed as having meanings consistent with the meanings herein and in related technical fields, shall not be construed as having idealized or excessively formal meanings.

Although terms such as first and second are sometimes employed to express various elements, it should be recognized that these elements are not to be limited by these terms. These terms are employed solely for the purpose of discriminating one element from another, and for example, to describe a first element as a second element, and similarly to describe that first element as the second element, is possible without departing from the scope of the present invention.

The present invention will now be more specifically described by Examples. However, the present invention can be embodied by various embodiments, and shall not be construed as being limited to the Examples described herein.

### Examples

### (Reference Example 1) Synthesis of disialo sugar chain asparagine mixture

To ethanol (EtOH, 67 mL) under stirring, one egg yolk was cracked and added. After stirring for about 5 hours, this was filtered, and further washed with EtOH (30 mL). To the precipitate obtained, EtOH (83 mL) was added again, and this was stirred overnight and then filtered, followed by washing of the precipitate with EtOH (30 mL). Next, the precipitate was dried to obtain 3 g of delipidated egg yolk.

After dissolving the delipidated egg yolk obtained in phosphate buffer (pH = 7.0, 30 mL), sodium azide (10 mg) was added. Further, Orientase ONS (from HBI, 1.0 g) was added, and this was left still at 50°C for about 24 hours. After confirming the completion of the reaction with thin layer chromatography (TLC), the reaction solution was filtered with Celite^{™} (Celite Corporation). After concentrating the filtrate, this was purified by gel filtration column chromatography (Sephadex G-25, 2.5 x 100 cm, water). The fraction containing the target sugar was collected and concentrated, followed by lyophilization.

To the dried powder obtained (about 430 mg) was added Tris-HCl/calcium chloride buffer (pH = 7.5, 43 mL) and sodium azide (21 mg) to dissolve the said powder. Further, Actinase E (43 mg) was added, and this was left still for 24 hours while checking every 12 hours that pH = 7.5. When a change in pH was confirmed, pH was adjusted to pH = 7.5 by aqueous trishydroxymethylaminomethane solution or aqueous hydrochloric acid solution. After leaving still for 24 hours, Actinase E (21.5 mg) was added again to the reaction solution, and this was allowed to react again for about 48 hours reaction while checking that pH = 7.5. When a change in pH was confirmed, pH was adjusted to pH = 7.5 by aqueous trishydroxymethylaminomethane solution or aqueous hydrochloric acid solution. After confirming the completion of the reaction with TLC, Celite filtration was performed. After concentrating the filtrate, this was purified by gel filtration column chromatography (Sephadex G-25, 2.5 x 100 cm, water). The fraction containing the target disialo sugar chain compound was collected and concentrated, followed by lyophilization, to obtain a disialo sugar chain asparagine mixture.

### (Reference Example 2) Synthesis of disialo sugar chain asparagine-Fmoc having amino group nitrogen of asparagine protected with Fmoc group

The disialo sugar chain asparagine mixture (120 mg) obtained in Reference Example 1 was dissolved in water (1.5 mL), and sodium hydrogen carbonate (26 mg) was added. Further, Fmoc-OSu [N-(9-Fluorenylmethyloxycarbonyl)oxysuccinimide] (68 mg) dissolved in dimethylformamide (DMF, 2.5 mL) was added, and then this was allowed to react at room temperature for 2 hours. After confirming the disappearance of the source material with TLC, this was concentrated with an evaporator. To the residue was added water (15 mL) and diethyl ether (25 mL), and this was stirred for 10 minutes. Next, after removing the diethyl ether layer, the aqueous layer was washed with diethyl ether (15 mL), and further, after concentration, lyophilization was performed. Next, the residue obtained was purified with an ODS column (Wakogel 100C18) on a gradient. The fraction containing the sugar chain was collected, and after concentration, lyophilization was performed. The residue obtained was purified by preparative HPLC column (YMC-Pack R&D ODS, D-ODS-5-A, 20 x 250 mm, acetonitrile/25 mM aqueous ammonium acetate solution = 20/80, 7.5 mL/min). After aliquoting the main peak that appears after about 15 minutes, this was concentrated. Next, desalting treatment was performed with an ODS column. This was lyophilized, and the target disialo sugar chain asparagine-Fmoc, which is a sugar chain asparagine compound having the amino group nitrogen of asparagine protected with Fmoc group, was obtained at 13.3 mg.

### (Reference Example 3) Preparation of (1S,2S(3)) disialo sugar chain asparagine-Fmoc (GT-25034)

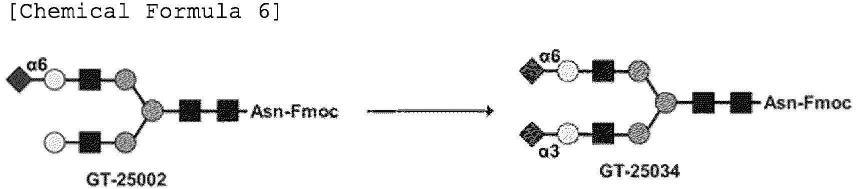

To GT-25002 (50 mg, MW: 2267.8, 22 µmol, GlyTech, Inc.), protease inhibitor (0.5 mL, 1 tablet of cOmplete, Roche, 11836170001 dissolved in water (0.5 mL)), 2 mM PMSF/DMSO (40 µL), Bacterial Alkaline Phosphatase (40 µL, Roche, 03359123001, 14 Units/µL), 1 M MOPS buffer (1.0 mL, pH = 7.0, NACALAI TESQUE, INC., 23442-81), BSA (50 mg, SIGMA, A3159-50G), and water (9.4 mL) were added, and 50 µL was dispensed for HPLC analysis. To the aqueous sugar chain solution after dispensation (about 11 mL), α2,3-sialyltransferase (291 mUnits, 40 Units/mL, JT, ish-467) and Cytidine 5'-monophospho-b-D-N-acetylneuraminic acid, disodium salt (CMP-sialic acid, 247 mg, from SANYO FINE CO., LTD.) were added, and this was shaken at 30°C. After 6 hours, 8 µL of the reaction solution was diluted in water (32 µL), and 5 µL was injected to perform HPLC analysis. Attenuation of the source material peak that elutes at 17.5 minutes in HPLC analysis, and production of a new peak that elutes at 15.5 minutes were confirmed. Mass spectrometry confirmed that the new peak produced was GT-25034, and the reaction was terminated. After termination of the reaction, and after purification by HPLC, concentration, and desalting by ODS support, (1S, 2S(3)) disialo sugar chain asparagine-Fmoc was obtained at 37 mg yield (MW: 2560.4, 15 µmol, yield 66%).

HPLC analysis condition (ODS): (CAPCELL PAK C18 UG120 250 x 4.6 mm, flow rate 0.70 mL/min, mobile phase A: 25 mM aqueous ammonium acetate solution, B: acetonitrile, gradient A 85% -> A 75% (20 min) -> A 5% (25 min) -> A 85% (45 min))

HPLC purification condition: (CAPCELL PAK C18 UG120 250 x 20 mm, flow rate 7.0 mL/min, mobile phase A: 25 mM aqueous ammonium acetate solution, B: acetonitrile, gradient A 80% -> A 80% (5 min) - > A 75% (25 min) -> A 10% (30 min) -> A 80% (45 min))

### (Reference Example 4) Preparation of ((1S(3),2S) disialo sugar chain asparagine-Fmoc (GT-25035)

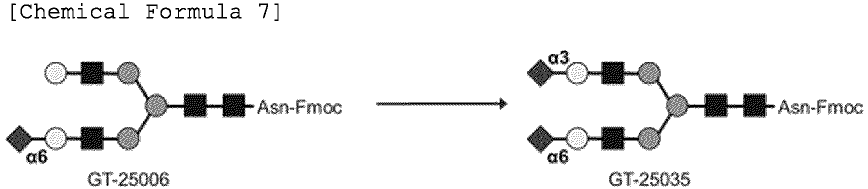

To GT-25006 (50 mg, MW: 2267.8, 22 µmol, GlyTech, Inc.), protease inhibitor (0.5 mL, 1 tablet of cOmplete, Roche, 11836170001 dissolved in water (0.5 mL)), 2 mM PMSF/DMSO (0.04 mL), Bacterial Alkaline Phosphatase (0.04 mL, Roche, 03359123001, 14 Units/µL), 1 M MOPS buffer (1.0 mL, pH = 7.0, NACALAI TESQUE, INC. 23442-81), BSA (50 mg, SIGMA, A3159-50G), and water (9.4 mL) were added, and 50 µL was dispensed for HPLC analysis. To the aqueous sugar chain solution after dispensation (about 11 mL), α2,3-sialyltransferase (291 mUnits, 40 Units/mL, JT, ish-467) and Cytidine 5'-monophospho-b-D-N-acetylneuraminic acid, disodium salt (CMP-sialic acid, 247 mg, from SANYO FINE CO., LTD.) were added, and this was shaken at 30°C. After 7 hours, 8 µL of the reaction solution was diluted in water (32 µL), and 5 µL was injected to perform HPLC analysis (analysis condition). Attenuation of the source material peak that elutes at 17.0 minutes in HPLC analysis, and production of a new peak that elutes at 14.5 minutes were confirmed. As a result of mass spectrometry, the new peak produced was confirmed to be GT-25035, and the reaction was terminated. After termination of the reaction, and after purification by HPLC, concentration, and desalting by ODS support, (1S, 2S(3)) disialo sugar chain asparagine-Fmoc yield 34.3 mg was obtained at (MW: 2560.35, 13.4 µmol, yield 61%).

HPLC analysis condition (ODS): (CAPCELL PAK C18 UG120 250 x 4.6 mm, flow rate 0.70 mL/min, mobile phase A: 25 mM aqueous ammonium acetate solution, B: acetonitrile, gradient A 85% -> A 75% (20 min) -> A 5% (25 min) -> A 85% (45 min))

HPLC purification condition: (CAPCELL PAK C18 UG120 250 x 20 mm, flow rate 7.0 mL/min, mobile phase A: 25 mM aqueous ammonium acetate solution, B: acetonitrile, gradient A 80% -> A 80% (5 min) - > A 75% (25 min) -> A 10% (30 min) -> A 80% (45 min))

### (Reference Example 5) Preparation of (1S(3),2S(3)) disialo sugar chain asparagine-Fmoc (GT-25025)

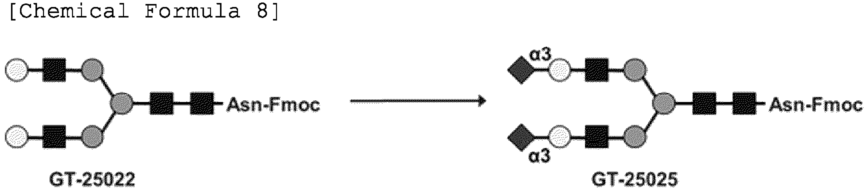

GT-25022 (5 g, MW: 1977.84, 2.5 mmol, GlyTech, Inc.) was dissolved in water (45 mL), and MOPS buffer (5.0 mL, pH = 7.0, NACALAI TESQUE, INC. 23442-81) was added, and then 200 µL was dispensed for HPLC analysis. To the aqueous sugar chain solution after dispensation (about 50 mL), Bacterial Alkaline Phosphatase (0.3 mL, Roche, 03359123001, 14 Units/µL), α2,3-sialyltransferase (500 mg) and Cytidine 5'-monophospho-b-D-N-acetylneuraminic acid, disodium salt (CMP-sialic acid, 5 g, from SANYO FINE CO., LTD.) were added, and this was shaken at 4°C. After 27 hours, 2 µL of the reaction solution was diluted in water (198 µL), and 10 µL was injected to perform HPLC analysis. Attenuation of the source material peak that elutes at 12.5 minutes in HPLC analysis, and production of a new peak that elutes at 8.0 minutes were confirmed. Mass spectrometry confirmed that the new peak produced was GT-25025, and the reaction was terminated. After termination of the reaction, and after purification by HPLC, concentration, and desalting by electrodialysis, (1S(3), 2S(3)) disialo sugar chain asparagine-Fmoc was obtained at 5.4 g yield (MW: 2560.35, 2.1 mmol, yield 83%).

HPLC analysis condition: (CAPCELL PAK C18 UG120 250 x 4.6 mm, flow rate 0.70 mL/min, mobile phase A: 25 mM aqueous ammonium acetate solution, B: acetonitrile, isocratic (A: 80%, B: 20%)

HPLC purification condition: (SP-300-5-ODS-BIO 250 x 30 mm, flow rate 25 mL/min, mobile phase A: 25 mM aqueous ammonium acetate solution, B: acetonitrile, gradient A 95% -> A 95% (10 min) -> A 85% (10.1 min) -> A 82% (40 min)

### (Example 1) Analysis of disialo sugar chain asparagine-Fmoc

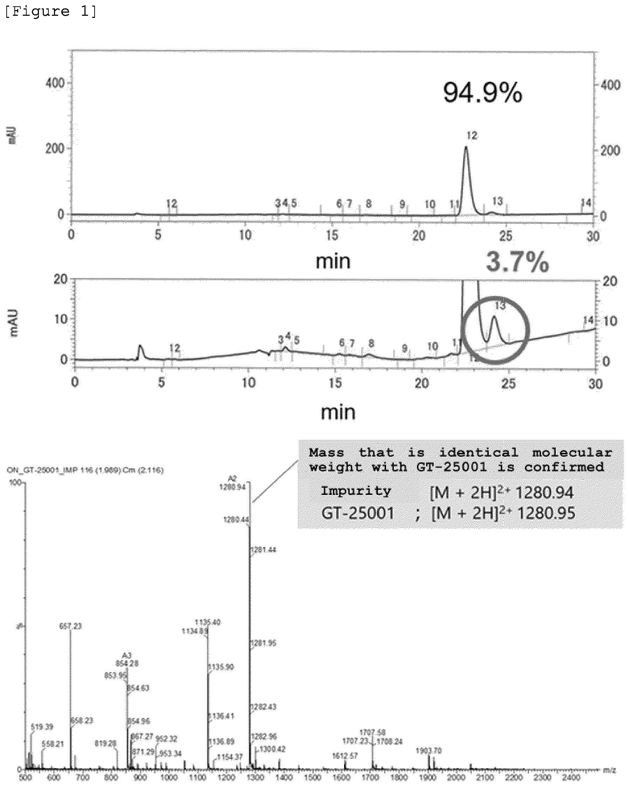

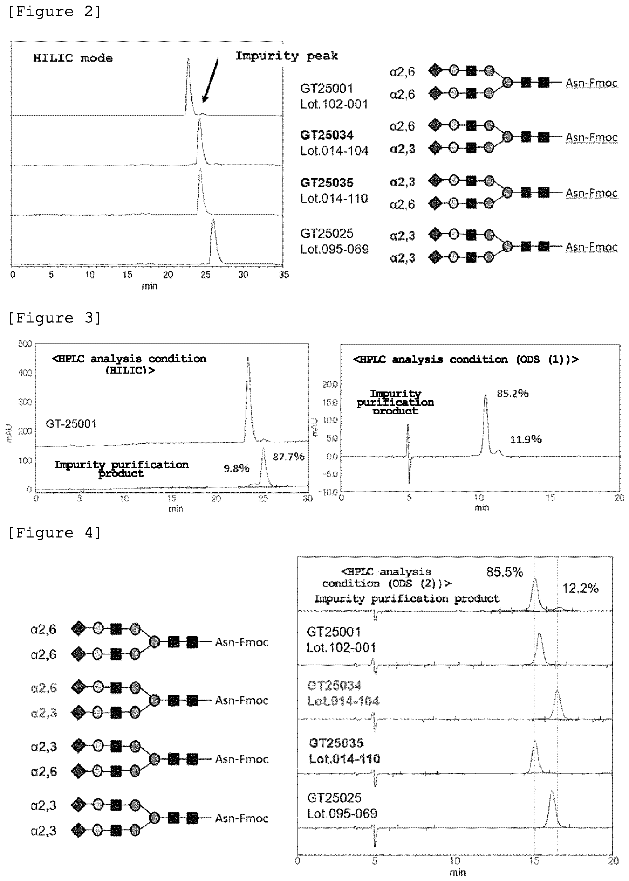

The disialo sugar chain asparagine-Fmoc obtained in Reference Example 2 (5 mg, 2.0 µmol) was dissolved in water, and filled up to 10.0 mL. The filled up aqueous sugar chain solution 10 µL was injected into HPLC and analyzed. As a result, after the disialo sugar chain asparagine-Fmoc peak (94.9%) that elutes at around 23 minutes, another peak showing the same mass that elutes at around 24 minutes (3.7%) was confirmed (Figure 1). As a result of comparing the retention time on HPLC with the α2,3-sialic acid-containing disialo sugar chains prepared in Reference Examples 3, 4, and 5, the peak that eluted at around 24 minutes was consistent with GT-25034 and GT-25035 (Figure 2).

HPLC analysis condition: (Asahipak NH2P-50 4E 5 µm 250 x 4.6 mm, flow rate 0.70 mL/min, mobile phase A: 0.5% aqueous phosphoric acid solution, B: 90% aqueous acetonitrile solution containing 0.5% phosphoric acid, gradient A 25% -> A 75% (25 min) -> A 90% (26 min) -> A 90% (28 min) -> A 25% (29 min) -> A 25% (45 min))

ESI-MS of peak that eluted at around 24 minutes: Calcd for C₁₀₃H₁₅₄N₈O₆₆[M+H]⁺ 2559.89, [M+2H]⁺² 1280.45, [M+3H]⁺³ 853.96, found 1280.44, 853.95.

### (Example 2) Impurity analysis of disialo sugar chain asparagine-Fmoc

After dissolving the disialo sugar chain asparagine-Fmoc obtained in Reference Example 2 (200 mg) in water (4 mL), this was diluted with acetonitrile (12 mL). After dilution, slightly clouded aqueous sugar chain solution was purified by HPLC and lyophilized. (HPLC purification condition) After lyophilization, by desalting with ODS support, impurity purification product (2.4 mg) was obtained (Figure 3).

As a result of comparative analysis of the impurity purification product obtained with sugar chains prepared by Reference Examples 2, 3, 4, and 5, GT-25034 and GT-25035 were each consistent (Figure 4).

HPLC purification condition: (Asahipak NH2P-90 20F 300 x 20 mm, flow rate 7 mL/min, mobile phase A: 0.5% aqueous phosphoric acid solution, B: 90% aqueous acetonitrile solution containing 0.5% phosphoric acid, gradient A 25% -> A 25% (5 min) -> A 75% (60 min)

HPLC analysis condition (ODS (1)): (CAPCELL PAK C18 UG120 250 x 4.6 mm, flow rate 0.70 mL/min, mobile phase A: 25 mM aqueous ammonium acetate solution, B: acetonitrile, isocratic (A: 80%, B: 20%)

HPLC analysis condition (ODS (2)): (CAPCELL PAK C18 UG120 250 x 4.6 mm, flow rate 0.70 mL/min, mobile phase A: 25 mM aqueous ammonium acetate solution, B: acetonitrile, isocratic (A: 82%, B: 18%)

HPLC analysis condition (HILIC): (Asahipak NH2P-50 4E 5 µm 250 x 4.6 mm, flow rate 0.70 mL/min, mobile phase A: 0.5% aqueous phosphoric acid solution, B: 90% aqueous acetonitrile solution containing 0.5% phosphoric acid, gradient A 25% -> A 75% (25 min) -> A 90% (26 min) -> A 90% (28 min) -> A 25% (29 min) -> A 25% (45 min))

### (Example 3) Estimate of structure of impurity

The impurity prepared in Example 2 was degraded by sialic acid hydrolase.

To the impurity (50 µg) dissolved in water (5 µL), 100 mM aqueous sodium acetate solution (44 µL) and α2,3-sialidase (5 mUnits, TAKARA, 4455) were added, and this was allowed to react at 37°C for 1.5 hours. After 1.5 hours, the reaction solution (10 µL) was diluted in water (90 µL), and 10 µL was injected to perform HPLC analysis. As a result of HPLC, the source material peaks at 10.5 minutes and 11.5 minutes had completely disappeared, and production of a new peak was confirmed at 14 minutes. As a result of mass spectrometry, it was confirmed that the new peak produced is a monosialo form having one sialic acid residue cleaved from the impurity.

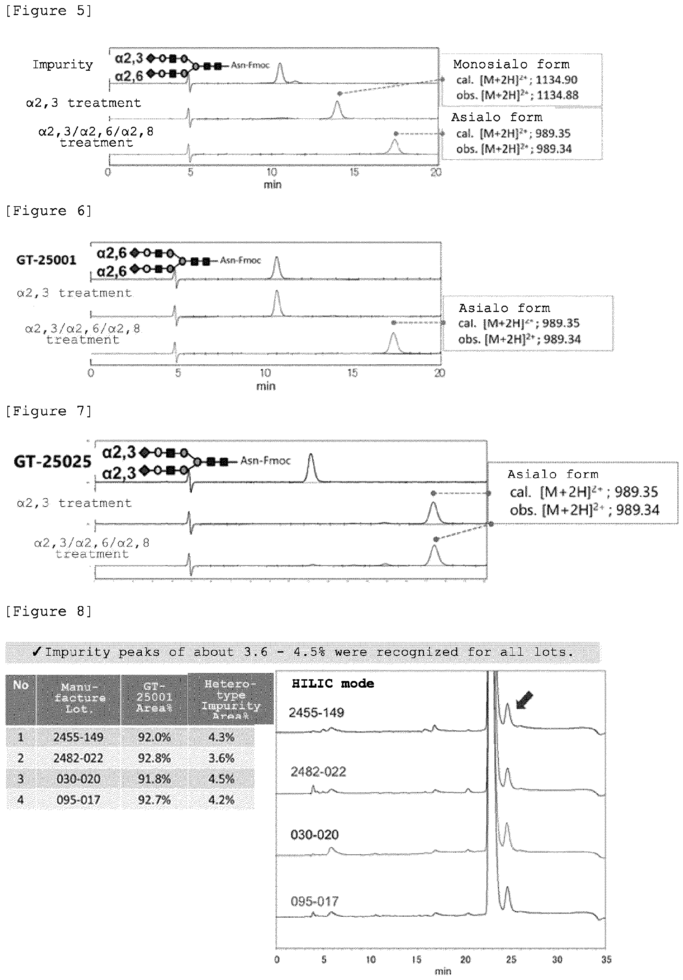

On the other hand, to the impurity (50 µg) similarly dissolved in water (5 µL), 100 mM aqueous sodium acetate solution (44 µL) and α2,3-2,6-2,8-sialidase (10 mUnits, Neuraminidase, SANYO FINE CO., LTD.) were added, and this was allowed to react at 37°C for 1.5 hours. After 1.5 hours, the reaction solution (10 µL) was diluted in water (90 µL), and 10 µL was injected to perform HPLC analysis. As a result of HPLC analysis, the source material peaks at 10.5 minutes and 11.5 minutes had completely disappeared, and production of a new peak was confirmed at 17.5 minutes. As a result of mass spectrometry, it was confirmed that the new peak produced is an asialo form having two sialic acid residues cleaved from the impurity (Figure 5).

HPLC analysis condition (ODS): (CAPCELL PAK C18 UG120 250 x 4.6 mm, flow rate 0.70 mL/min, mobile phase A: 25 mM aqueous ammonium acetate solution, B: acetonitrile isocratic (A: 80%, B: 20%)

### (Example 4) Analysis of enzyme digestion product of GT-25001

GT-25001 was degraded by sialic acid hydrolase.

To GT-25001 (50 µg) dissolved in water (5 µL), 100 mM aqueous sodium acetate solution (44 µL) and α2,3-sialidase (5 mUnits, TAKARA, 4455) were added, and this was allowed to react at 37°C for 1.5 hours. After 1.5 hours, the reaction solution (10 µL) was diluted in water (90 µL), and 10 µL was injected to perform HPLC analysis. As a result of HPLC analysis, it was confirmed that there is no change from the source material peak at 11 minutes (Figure 6).

On the other hand, to GT-25001 (50 µg) similarly dissolved in water (5 µL), 100 mM aqueous sodium acetate solution (44 µL) and α2,3-2,6-2,8-sialidase (10 mUnits, Neuraminidase, SANYO FINE CO., LTD.) were added, and this was allowed to react at 37°C. After 1.5 hours, the reaction solution (10 µL) was diluted in water (90 µL), and 10 µL was injected to perform HPLC analysis. As a result of HPLC analysis, the source material peak at 11 minutes had completely disappeared, and production of a new peak was confirmed at 17.5 minutes. As a result of mass spectrometry, it was confirmed that the new peak produced is an asialo form having two sialic acid residues cleaved from GT-25001 (Figure 6).

HPLC analysis condition (ODS): (CAPCELL PAK C18 UG120 250 x 4.6 mm, flow rate 0.70 mL/min, mobile phase A: 25 mM aqueous ammonium acetate solution, B: acetonitrile, isocratic (A: 80%, B: 20%)

### (Example 5) Analysis of enzyme digestion product of GT-25025

GT-25025 was degraded by sialic acid hydrolase.

To GT-25025 (50 µg) dissolved in water (5 µL), 100 mM aqueous sodium acetate solution (44 µL) and α2,3-sialidase (5 mUnits, TAKARA, 4455) were added, and this was allowed to react at 37°C for 1.5 hours. After 1.5 hours, the reaction solution (10 µL) was diluted in water (90 µL), and 10 µL was injected to perform HPLC analysis. As a result of HPLC analysis, the source material peak at 11 minutes had completely disappeared, and production of a new peak was confirmed at 17.5 minutes. As a result of mass spectrometry, it was confirmed that the new peak produced is an asialo form having two sialic acid residues cleaved from GT-25025 (Figure 7).

On the other hand, to GT-25025 (50 µg) similarly dissolved in water (5 µL), 100 mM aqueous sodium acetate solution (44 µL) and α2,3-2,6-2,8-sialidase (10 mUnits, Neuraminidase, SANYO FINE CO., LTD.) were added, and this was allowed to react at 37°C for 1.5 hours. After 1.5 hours, the reaction solution (10 µL) was diluted in water (90 µL), and 10 µL was injected to perform HPLC analysis. As a result of HPLC analysis, the source material peak at 11 minutes had completely disappeared, and production of a new peak was confirmed at 17.5 minutes. As a result of mass spectrometry, it was confirmed that the new peak produced is an asialo form having two sialic acid residues cleaved from GT-25025 (Figure 7).

HPLC analysis condition (ODS): (CAPCELL PAK C18 UG120 250 x 4.6 mm, flow rate 0.70 mL/min, mobile phase A: 25 mM aqueous ammonium acetate solution, B: acetonitrile, isocratic (A: 80%, B: 20%)

### (Example 6) Analysis of difference between lots of disialo sugar chain asparagine mixture obtained from chicken egg

From multiple production lots of delipidated egg yolk as the source material, disialo sugar chain asparagine-Fmocs were prepared according to Reference Examples 1 and 2. The disialo sugar chain asparagine-Fmoc obtained (5 mg, 2.0 µmol) was dissolved in water, and filled up to 10.0 mL. The filled up aqueous sugar chain solution 10 µL was injected into HPLC and analyzed. As a result, it was confirmed that all lots comprise the peak that elutes at around 24 minutes corresponding to impurities GT-25034 and GT-25035 in the range of between about 3.5% and 5.0% (Figure 8).

HPLC analysis condition: (Asahipak NH2P-50 4E 5 µm 250 x 4.6 mm, flow rate 0.70 mL/min, mobile phase A: 0.5% aqueous phosphoric acid solution, B: 90% aqueous acetonitrile solution containing 0.5% phosphoric acid, gradient A 25% -> A 75% (25 min) -> A 90% (26 min) -> A 90% (28 min) -> A 25% (29 min) -> A 25% (45 min))

### (Example 7) Purification of disialo sugar chain asparagine-Fmoc

The disialo sugar chain asparagine-Fmoc obtained in Reference Example 2 (50 mg, 20 µmol) was dissolved in 75% acetonitrile solution (2.5 mL). This was injected to an HPLC column, and purification was performed so as not to allow contamination by impurities GT-25034 and GT-25035. By desalting with the fraction comprising GT-25001, GT-25001 (36 mg, 14 µmol, yield 70%) having contamination of GT-25034 and GT-25035 at 0.2% is obtained.

Preparative HPLC condition: (Asahipak NH2P-90 20F 300 x 20 mm, flow rate 8 mL/min, mobile phase A: 5% acetic acid/3% triethylamine-containing aqueous solution, B: 2% acetic acid-containing acetonitrile solution, gradient A 25% -> A 25% (5 min) -> A 65% (5.1 - 65 min)

### (Example 8) Removal of impurity contained in disialo sugar chain asparagine-Fmoc mixture (1)

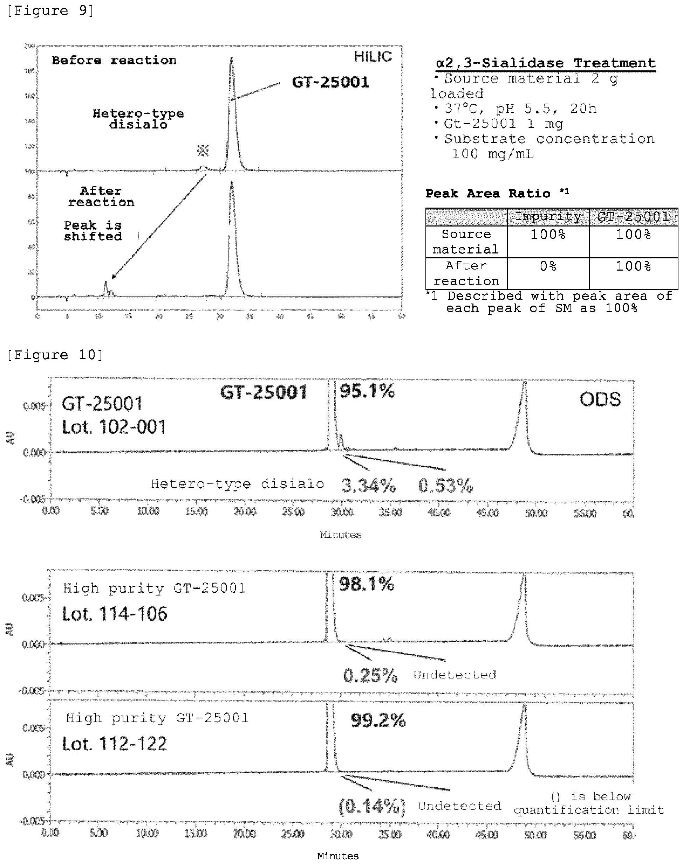

The disialo sugar chain asparagine-Fmoc obtained in Reference Example 2 (2 g) was dissolved in a buffer (pH = 5.5, 20 mL) comprising 100 mM sodium acetate and 10 mM calcium chloride, α2,3-sialidase (2 Units, TAKARA, 4455) was added, and this was allowed to react at 37°C. After 16 hours, the reaction solution (1 µL) was diluted in water (50 µL), and then acetonitrile (50 µL) was added, and 20 µL was injected to perform HPLC analysis. As a result of HPLC analysis, the peak at 32 minutes (GT-25001) did not change, the peak at 27 minutes (GT-25034/GT-25035 mixture) had completely disappeared, and production of a new peak was confirmed at 11 minutes. As a result of mass spectrometry, it was confirmed that the new peak produced is a monosialo form having one sialic acid residue cleaved from GT-25034 and GT-25035 (Figure 9).

After confirming the completion of the reaction, the reaction solution was heated at 80°C for 10 minutes. The enzyme treatment solution obtained was purified by an HPLC column, and by desalting the fraction comprising GT-25001 with ODS support, GT-25001 (1.4 g, yield 70%) having GT-25034/GT-25035 mixture at 0.3% or less was obtained (Figure 10).

HPLC analysis condition: (Asahipak NH2P-50 4E 5 µm 250 x 4.6 mm, flow rate 0.70 mL/min, mobile phase A: 5% acetic acid, 3% triethylamine-containing aqueous solution, B: 2% acetic acid-containing acetonitrile, isocratic (A: 60%, B: 40%)

Preparative HPLC condition: (SP-300-5-ODS-BIO 5 µm 250 x 30 mm, flow rate 25 mL/min, mobile phase A: 25 mM aqueous ammonium acetate solution, B: acetonitrile, gradient A 90% -> A 80% (30 min)

ESI-MS: Calcd for C₁₀₃H₁₅₉N₈O₆₆[M+H]⁺ 2559.89, [M+2H]⁺² 1280.45, [M+3H]⁺³ 853.96, found 1280.44, 853.95.

### (Example 9) Removal of impurity contained in disialo sugar chain asparagine-Fmoc (2) (derivatization by lactonization)

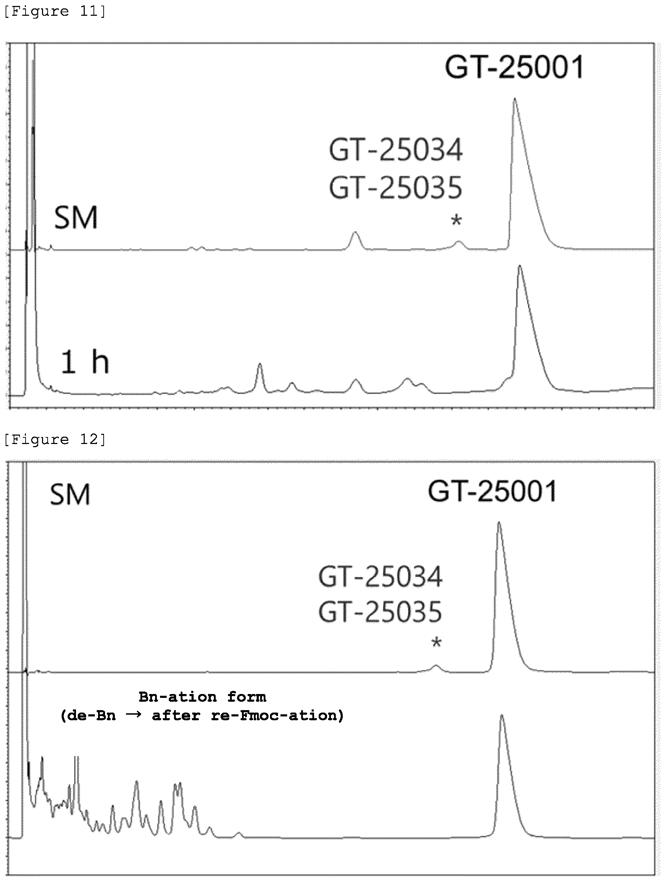

To the disialo sugar chain asparagine-Fmoc obtained in Reference Example 2 (50 mg), 500 mM HOBt and 500 mM DIC were added, and this was allowed to react at room temperature for 1 hour. After 1 hour, the reaction solution (10 µL) was diluted with acetonitrile/water mixed solution (7:3, 90 µL), and then 5 µL was injected to perform HPLC analysis. As a result of HPLC analysis, it was confirmed that the peak at 28 minutes (GT-25001) did not largely change, and the peak at 24 minutes (GT-25034/GT-25035 mixture) had completely disappeared (Figure 11).

Preparative HPLC condition: (CAPCELL PAK UG120 C18 5 µm 250 x 4.6 mm, flow rate 0.7 mL/min developing solvent A: water/trifluoroacetic acid (1000:1), B: acetonitrile/trifluoroacetic acid (1000:1) gradient A 99% -> A 70% (30 min)

HPLC analysis condition: (ACQUITY UPLC Glycan BEH Amide, 1.7 µm 150 x 2.1 mm, flow rate 0.35 mL/min, mobile phase A: 50 mM formic acid ammonium (pH 4.4), B: acetonitrile, gradient A 30% -> A 30% (66 min)

### (Example 10) Removal of impurity contained in disialo sugar chain asparagine-Fmoc (3) (derivatization (esterification) by benzyl group)

To the disialo sugar chain asparagine-Fmoc obtained in Reference Example 2 (25 mg), NMP/DMF/water (84:15:1) (250 µL) was added, and this was stirred at 40°C for 1 hour to dissolve. After 1 hour, benzyl bromide (8.6 µL, 3.6 equivalents) was added, and this was stirred at 40°C. After 6 hours, 1 M sodium carbonate (50 µL) was added, and this was stirred at room temperature for 2 hours, and then purified by an ODS column. After desalting by solid-phase extraction, lyophilization was performed, and GT-25001 having sialic acid Bn-ated was obtained at 2.5 mg. In order to confirm the removal of impurities, to GT-25001 having the sialic acid Bn-ated obtained (1 mg), 100 mM sodium hydroxide (10 µL) was added, this was allowed to react at 40°C for 1 hour, and the Bn and Fmoc groups were deprotected. Subsequently, 1 M sodium hydrogen carbonate (10 µL), acetonitrile (20 µL), and Fmoc-OSu (2.4 mg) were added for re-Fmoc-ation. After subjecting this solution to solid-phase extraction to remove the contaminant, this was concentrated under reduced pressure. Water was added to the concentrated solution to obtain a sample solution. As a result of analyzing the sample solution, it was confirmed that the peak at 24 minutes (GT-25034/GT-25035 mixture) had completely disappeared (Figure 12).

Preparative HPLC condition: (CAPCELL PAK UG120 C18 5 µm 250 x 20 mm, flow rate 7.0 mL/min developing solvent A: 25 mM ammonium acetate, B: acetonitrile gradient A 75% -> A 70% (30 min)

HPLC analysis condition: (ACQUITY UPLC Glycan BEH Amide, 1.7 µm 150 x 2.1 mm, flow rate 0.35 mL/min, mobile phase A: 50 mM formic acid ammonium (pH 4.4), B: acetonitrile, gradient A 30% -> A 30% (66 min)

### (Example 11) Removal of impurity contained in SGP (derivatization by 2,3-sialidase)

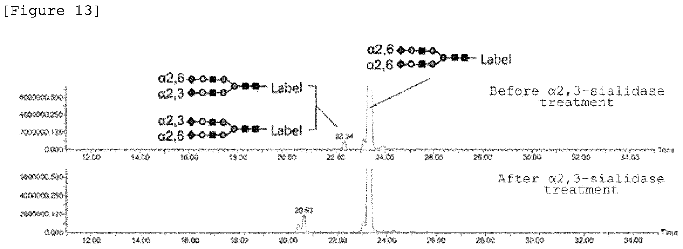

SGP (from TCI, S0523, 2 mg, 1.6 µmol) was dissolved in 100 mM sodium acetate buffer (pH 5.5), 2 µL of α2,3-sialidase (0.5 U/mU, TAKARA, 4455) was added, and this was reacted at 37°C for 2 hours and purified, to obtain SGP with the impurity removed (0.6 mg, yield 33%). Water was added to the SGP with the impurity removed to adjust to 2 mg/mL, to obtain an aqueous SGP solution. To the aqueous SGP solution (7.5 µL) was added water (15.3 µL) and 5% RapiGest solution (6 µL), and this was incubated at 90°C for 3 minutes. The solution was returned to room temperature, Rapid PNGase (1.2 µL) was added, and this was incubated at 50°C for 3 minutes. To this solution was added GlycoWorks RapiFluor-MS labeling reagent (12 µL), and this was left still at room temperature for 5 minutes. To this solution was added acetonitrile (358 µL) for dilution, RFMS labeling sugar chain was solid-phase extracted with GlycoWorks HILIC µElution Plate, and this was lyophilized and then redissolved in the solvent to obtain a sample solution. As a result of analyzing the sample solution, the disappearance of impurities derived from α2,6/α2,3 and α2,3/α2,6 was confirmed (Figure 13).

Preparative HPLC condition: (CAPCELL PAK UG120 C18 5 µm 250 x 4.6 mm, flow rate 0.7 mL/min developing solvent A: water/trifluoroacetic acid (1000:1), B: acetonitrile/trifluoroacetic acid (1000:1) gradient A 99% -> A 70% (30 min)

HPLC analysis condition: (ACQUITY UPLC Glycan BEH Amide, 1.7 µm 150 x 2.1 mm, flow rate 0.35 mL/min, mobile phase A: 50 mM formic acid ammonium (pH 4.4), B: acetonitrile, gradient A 25% -> A 31% (66 min)

## Claims

1. A method of manufacturing a composition comprising sugar chains having α2,6-linked sialic acids at all non-reducing ends at high purity from a chicken egg-derived sugar chain extract, **characterized by** performing a treatment for enriching sugar chains having α2,6-linked sialic acids at all non-reducing ends in said chicken egg-derived sugar chain extract.

2. The manufacturing method according to claim 1, wherein said sugar chain is a disialo sugar chain.

3. The manufacturing method according to claim 1, wherein said sugar chain is in the form of the following compound:

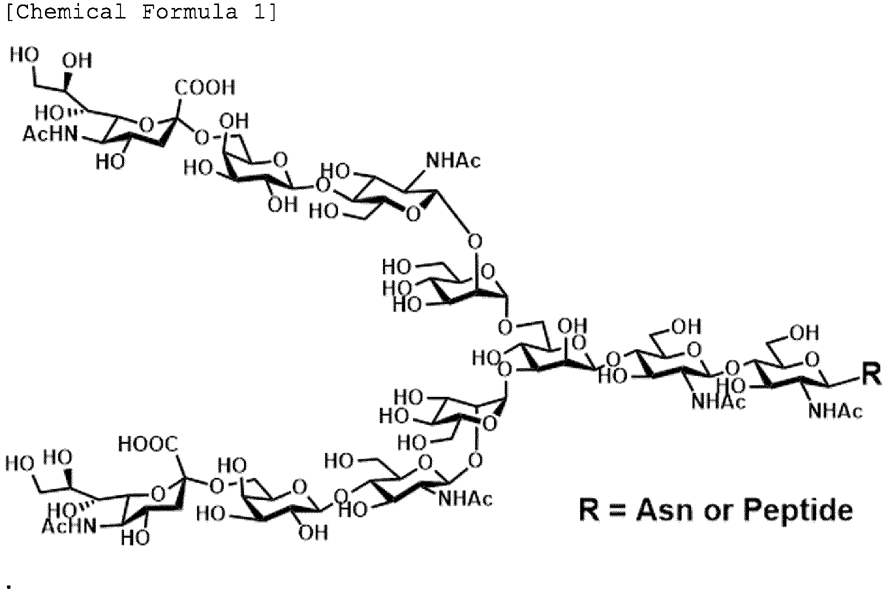

4. The manufacturing method according to claim 1, wherein said enrichment treatment comprises separating sugar chains having α2,6-linked sialic acids at all non-reducing ends from sugar chains having α2,3-linked sialic acid at any non-reducing end.

5. The manufacturing method according to claim 4, wherein said separation is performed by liquid chromatography.

6. The manufacturing method according to claim 1, wherein said enrichment treatment comprises performing enzyme treatment or treatment by acid hydrolysis on sugar chains having α2,3-linked sialic acid at any non-reducing end.

7. The manufacturing method according to claim 6, wherein said enzyme treatment comprises treatment by α2,3-sialidase.

8. The manufacturing method according to claim 1, wherein said enrichment treatment comprises derivatization of sugar chains having α2,6-linked sialic acids at all non-reducing ends or sugar chains having α2,3-linked sialic acid at any non-reducing end.

9. The manufacturing method according to claim 1, further comprising introducing a lipophilic substituent into the sugar chain.

10. The manufacturing method according to claim 9, wherein the introduction method of the lipophilic substituent is esterification, thioesterification, amidation, or hydrazidation of the sialic acid carboxy group.

11. The manufacturing method according to claim 9, wherein the lipophilic substituent is a benzyl group introduced into the carboxylic acid of sialic acid.

12. A composition manufactured by the manufacturing method according to any one of claims 1 to 11.

13. A composition consisting essentially of sugar chains having α2,6-linked sialic acids at all non-reducing ends.

14. The composition according to claim 13, wherein said sugar chain is a disialo sugar chain.

15. The manufacturing method according to claim 13, wherein said sugar chain is in the form of the following compound:

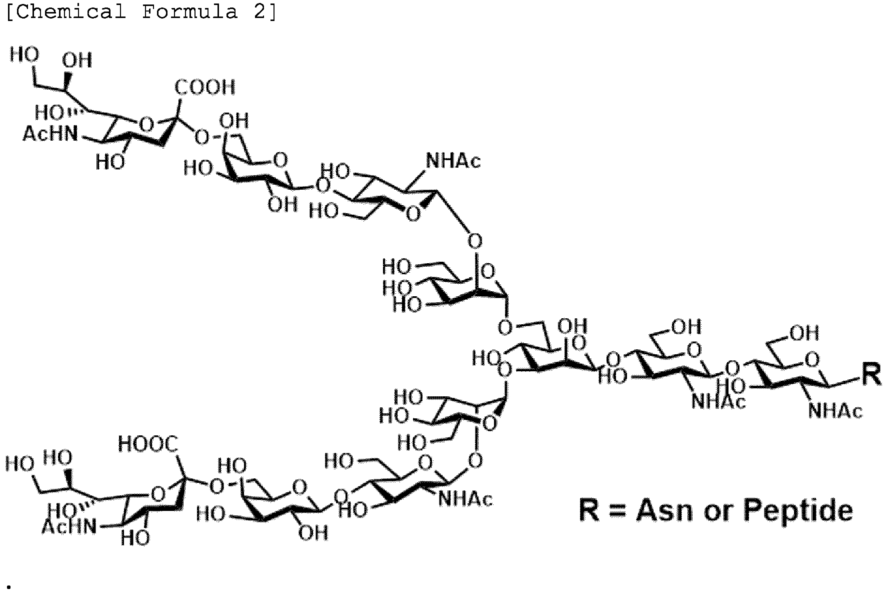

16. The composition according to claim 13, wherein at least 97% of all sugar chains are sugar chains having α2,6-linked sialic acids at all non-reducing ends.

17. The composition according to claim 13, wherein at least 98% of all sugar chains are sugar chains having α2,6-linked sialic acids at all non-reducing ends.

18. The composition according to claim 13, wherein at least 99% of all sugar chains are sugar chains having α2,6-linked sialic acids at all non-reducing ends.

19. The composition according to claim 13, wherein
at least 95% of all sugar chains are sugar chains having α2,6-linked sialic acids at all non-reducing ends, and
it comprises sugar chains having α2,3-linked sialic acid at any non-reducing end at less than 2% of all sugar chains.

20. A method of manufacturing a composition comprising sugar chains having α2,6-linked sialic acids at all non-reducing ends at high purity from a sugar chain mixture comprising sugar chains having α2,6-linked sialic acids at all non-reducing ends and sugar chains having α2,3-linked sialic acid at any non-reducing end, **characterized by** purifying said sugar chains in said sugar chain mixture by the following:
(a) subjecting to liquid chromatography employing a HILIC column
(b) degrading or denaturing sugar chains having α2,3-linked sialic acid on any of the non-reducing ends, and then subjecting to liquid chromatography; or
(c) derivatizing sugar chains having α2,6-linked sialic acids at all non-reducing ends, and then subjecting to liquid chromatography.

21. The manufacturing method according to claim 20, wherein said sugar chain is a disialo sugar chain.

22. The manufacturing method according to claim 20, wherein said sugar chain is in the form of the following compound:

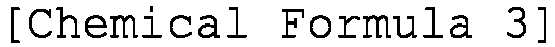

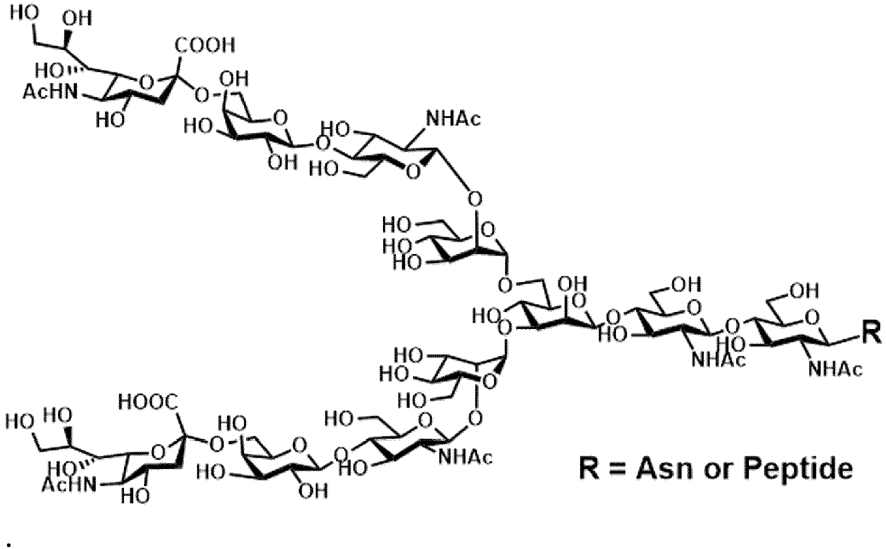

23. The manufacturing method according to claim 20, wherein said degradation comprises performing enzyme treatment or treatment by acid hydrolysis on sugar chains having α2,3-linked sialic acid at any non-reducing end.

24. The manufacturing method according to claim 23, wherein said enzyme treatment comprises treatment by α2,3-sialidase.

25. The manufacturing method according to claim 20, wherein said denaturing comprises derivatizing any of sugar chains having α2,6-linked sialic acids at all non-reducing ends and sugar chains having α2,3-linked sialic acid at any of the non-reducing ends, or both.
